# EUROPÄISCHE PATENTSCHRIFT

(11) **EP 0 640 104 B1**
(45) Veröffentlichungstag und Bekanntmachung des Hinweises auf die Patenterteilung: **04.12.1996**
(21) Anmeldenummer: 93909900.8
(22) Anmeldetag: 06.05.1993
(51) Int. Cl.: C08F 222/06, C08F 222/14, A61K 7/00

(54) **COPOLYMERISATE AUF BASIS VON VINYLETHERN UND MONOETHYLENISCH UNGESÄTTIGTEN DICARBONSÄUREANHYDRIDEN, VERFAHREN ZU IHRER HERSTELLUNG UND IHRE VERWENDUNG**
COPOLYMERS BASED ON VINYL ETHERS AND MONOETHYLENICALLY UNSATURATED DICARBOXYLIC ACID ANHYDRIDES, PROCESS FOR PRODUCING THE SAME AND THEIR USE
COPOLYMERISATS A BASE D'ETHERS VYNILIQUES ET D'ANHYDRIDES D'ACIDE DICARBOXYLIQUES MONOETHYLENIQUEMENT INSATURES, LEUR PROCEDE DE FABRICATION ET LEUR UTILISATION

(30) Priorität: 16.05.1992 DE 4216318
(43) Veröffentlichungstag der Anmeldung: 01.03.1995
(73) Patentinhaber: BASF Aktiengesellschaft, 67063 Ludwigshafen (DE)
(72) Erfinder: MEYER, Harald, Dr., D-67157 Wachenheim (DE); DENZINGER, Walter, D-6720 Speyer (DE); SANNER, Axel, D-6710 Frankenthal (DE); RICHTER, Hans, D-6700 Ludwigshafen (DE); RAUBENHEIMER, Hans-Juergen, D-6834 Ketsch (DE); FROSCH, Franz, D-6702 Bad Duerkheim (DE)
(86) Internationale Anmeldenummer: EP9301103
(87) Internationale Veröffentlichungsnummer: WO9323445

(56) Entgegenhaltungen:
- EP-A- 0 428 956
- EP-A- 0 461 489
- US-A- 3 436 378

## Beschreibung

Die Erfindung betrifft Copolymerisate aus Vinylalkylethern, monoethylenisch ungesättigten Dicarbonsäureanhydriden und Dialkylestern von monoethylenisch ungesättigten Dicarbonsäuren, Verfahren zu ihrer Herstellung und ihre Verwendung als filmbildende Harze in Haarsprays oder in Form der wasserlöslichen Salze als Verdickungsmittel für wäßrige Systeme.

Aus der FR-A-1 527 411 sind Copolymerisate bekannt, die aus Maleinsäureanhydrid, Alkenen und Vinylalkylethern bestehen und durch Fällungspolymerisation der Monomeren, beispielsweise in Benzol, in Gegenwart von Radikalen bildenden Polymerisationsinitiatoren erhältlich sind. Die Anhydridgruppe der Copolymerisate kann nach der Copolymerisation hydrolisiert und gegebenenfalls neutralisiert werden.

Aus der BE-A-710 985 sind Terpolymerisate bekannt, die aus Vinylalkylethern, Maleinsäureanhydrid und einem dritten Monomeren, wie beispielsweise Acrylsäure, Methacrylsäure, Acrylamid, Methacrylamid, Vinylchlorid oder Styrol aufgebaut sind. Die Terpolymerisate werden in der Papierindustrie verwendet. Aus der NE-A 68/10081 ist bekannt, daß man Copolymerisate aus a) Maleinsäureanhydrid, b) Vinylalkylethern, Ethylen-, Propylen- oder Vinylacetat und c) 1-Alkenen oder langkettigen Vinylalkylethern in inerten Lösemittel herstellen kann.

Aus der EP-A-0428956 ist ein Verfahren zur Herstellung von Copolymerisaten aus Maleinsäuremonoalkylestern, Vinylalkylethern und Maleinsäureanhydrid bekannt. Bei diesem Verfahren wird ein Überschuß an Vinylalkylether als Löse- und Fällungsmittel verwendet. Die spezifische Viskosität bzw. die Kettenlänge der Copolymerisate wird über das Verhältnis von Maleinsäuremonoalkylester zu Maleinsäureanhydrid gesteuert.

Aus der US-A-5 064 897 ist die Herstellung von Copolymeren aus Monoalkylmaleaten und Vinylalkylethern bekannt. Dabei wird ein sechs- bis zehnfacher Überschuß an Vinylalkylethern eingesetzt und das monomere Monoalkylmaleat direkt vor der Polymerisation durch Reaktion von Alkoholen mit Maleinsäureanhydrid in äquimolarem Verhältnis hergestellt.

Die Umsetzung von Maleinsäureanhydrid-Einheiten enthaltenden Polymerisaten mit Alkoholen in einer polymeranalogen Reaktion ist beispielsweise aus folgenden Literaturstellen bekannt: JP-A-1983/25 982, GB-A-1 233 468, DE-A-1 930 009, EP-A-03 10 079 und EP-A-04 61 489.

Der vorliegenden Erfindung liegt die Aufgabe zugrunde, neue Polymerisate sowie ein Verfahren zu ihrer Herstellung zur Verfügung zu stellen.

Die zuerst genannte Aufgabe wird gelöst mit Copolymerisaten auf Basis von Vinylalkylethern und monoethylenisch ungesättigten Dicarbonsäurenanhydriden, wenn die Copolymerisate
a) 2 bis 50 Mol.-% Vinylalkylether mit 3 bis 30 C-Atomen im Molekül,
b) 10 bis 88 Mol.-% monoethylenisch ungesättigte Dicarbonsäureanhydride und
c) 10 bis 88 Mol.-% Dialkylester von monoethylenisch ungesättigten Dicarbonsäuren
copolymerisiert enthalten. Die andere Aufgabe wird mit einem Verfahren zur Herstellung der Copolymerisate gelöst, wenn man
a) 2 bis 50 Mol.-% Vinylalkylether mit 3 bis 30 C-Atomen im Molekül,
b) 10 bis 88 Mol.-% monoethylenisch ungesättigte Dicarbonsäureanhydride und
c) 10 bis 88 Mol.-% Dialkylester von monoethylenisch ungesättigten Dicarbonsäuren
bei Temperaturen von 40 bis 180°C in Gegenwart von Initiatoren copolymerisiert, die unter den Bedingungen der Copolymerisation Radikale bilden. Die so erhältlichen Copolymerisate werden als filmbildende Harze in Haarsprays oder in Form der Alkalimetall-, Ammonium-, oder Erdalkalimetallsalze als Verdickungsmittel für wäßrige Systeme verwendet.

Die Copolymerisate enthalten als Monomere der Komponente a) Vinylalkylether mit 3 bis 30 C-Atomen im Molekül. Geeignete Monomere dieser Art sind beispielsweise Vinylmethylether, Vinylethylether, Vinylisopropylether, Vinyl-n-propylether, Vinyl-n-butylether, Vinylisobutylether, Vinyloctylether, Vinyl-n-hexadecylether, Vinyl-n-octadecylether, Vinyl-n-eicosylether, Vinyl-n-hexacosylether und Vinyl-n-tricontylether. Die Copolymerisate können entweder einen einzigen Vinylether oder eine Mischung mehrerer Vinylether einpolymerisiert enthalten. Bevorzugt enthalten die Copolymerisate als Monomere der Gruppe a) Vinylmethylether, Vinylethylether oder Vinyloctadecylether. Die Monomeren der Gruppe a) sind zu 2 bis 50, vorzugsweise 25 bis 50 mol.-% am Aufbau der Copolymerisate beteiligt.

Die Copolymerisate enthalten als Monomer der Gruppe b) monoethylenisch ungesättigte Dicarbonsäureanhydride, die sich vorzugsweise von Verbindungen mit 4 bis 8 Kohlenstoffatomen ableiten. Geeignete Verbindungen dieser Art sind beispielsweise Maleinsäureanhydrid, Itaconsäureanhydrid, Glutaconsäureanhydrid, Methylenmalonsäureanhydrid, Citraconsäureanhydrid und Mischungen der genannten Anhydride. Die Copolymerisate enthalten bevorzugt Maleinsäureanhydrideinheiten. Die Menge der in die Copolymerisate einpolymerisierten Monomeren der Gruppe b) beträgt 10 bis 88, vorzugsweise 20 bis 60 mol.-%.

Als weitere charakteristische Monomere enthalten die Copolymerisate c) Dialkylester von monoethylenisch ungesättigten Dicarbonsäuren einpolymerisiert. Die Alkylgruppe der Ester kann sich von C₁- bis C₃₀-Alkoholen ableiten. Die Säurekomponente der Ester wird vorzugsweise von monoethylenisch ungesättigten Dicarbonsäuren mit 4 bis 8 C-Atomen gebildet. Beispiele für die Monomeren c) sind Dialkylester von Maleinsäure, Fumarsäure, Itaconsäure, Glutaconsäure, Methylenmalonsäure und Citraconsäure. Die beiden Alkylreste der Dialkylester können dabei entweder gleich oder verschieden sein und beispielsweise eine Methyl-, Ethyl-, n-Propyl-, Isopropyl-, n-Butyl-, Isobutyl-, sec.-Butyl-, n-Amyl-, n-Hexyl-, n-Octyl- oder n-Octadecylgruppen bedeuten. Die Monomeren der Gruppe c) können entweder alleine oder in Mischung untereinander in copolymerisierter Form im Copolymerisat vorliegen. Die Copolymerisate enthalten bevorzugt Diethyl- oder Di-n-Butylester der Maleinsäure einpolymerisiert. Die Monomeren der Gruppe c) sind zu 10 bis 88, vorzugsweise 20 bis 60 mol.-% am Aufbau der Copolymerisate beteiligt.

Die Copolymerisate werden durch Copolymerisieren der Monomeren der Gruppen a), b) und c) bei Temperaturen von 40 bis 180°C in Gegenwart von Initiatoren hergestellt, die unter den Bedingungen der Copolymerisation Radikale bilden. Die Copolymerisation kann nach allen bekannten Polymerisationstechniken vorgenommen werden, z. B. als Lösungs- oder Fällungspolymerisation. Geeignete inerte Löse- bzw. Fällungsmittel, die nicht mit den Monomeren a) bis c) unter den Bedingungen der Copolymerisation reagieren, sind Ketone, wie Aceton, Methylethylketon oder Diethylketon, aromatische Verbindungen wie Benzol, Toluol oder Xylol, Ester wie Ethylacetat, Isopropylacetat, Isobutylacetat oder n-Butylacetat, geradkettige und verzweigte aliphatische oder cycloaliphatische Kohlenwasserstoffe wie Pentan, Hexan, Heptan, Octan, Isooctan, Cyclohexan, Diethylcyclohexan und Dimethylcyclohexan.

Besonders bevorzugt werden die Copolymerisate jedoch durch Copolymerisation in Masse hergestellt, d. h. bei dieser Verfahrensweise wird auf die Anwesenheit inerter Löse- oder Verdünnungsmittel verzichtet. Die Monomeren können dabei zu Beginn der Copolymerisation in der Polymerisationsvorrichtung vorgelegt werden oder aber - diese Verfahrensweise ist bevorzugt - während der Copolymerisation portionsweise oder kontinuierlich dem Polymerisationsreaktor zugeführt werden. Die Polymerisationstemperatur liegt bevorzugt in dem Bereich von 50 bis 140°C. Die Polymerisation wird üblicherweise bei einer Temperatur durchgeführt, bei der die Reaktionsmischung flüssig ist, damit das Reaktionsgemisch problemlos gerührt werden kann. Die Polymerisationsdauer beträgt etwa 1 Stunde bis etwa 15 Stunden und ist meistens innerhalb einer Zeit von 2 bis 9 Stunden beendet. Die Copolymerisation kann bei Normaldruck, unter vermindertem Druck, z. B. bei 100 mbar oder erhöhtem Druck durchgeführt werden, z. B. bis zu Drükken von 50 bar und darüber.

Initiatoren, die unter den Bedingungen der Copolymerisation Radikale bilden, sind vorzugsweise alle diejenigen Verbindungen geeignet, die bei der jeweils gewählten Polymerisationstemperatur eine Halbwertszeit von weniger als 3 Stunden aufweisen. Falls man die Polymerisation zunächst bei niedriger Temperatur startet und bei höherer Temperatur zu Ende führt, ist es zweckmäßig, mit mindestens zwei bei verschiedenen Temperaturen zerfallenden Initiatoren zu arbeiten, nämlich zunächst einen beireits bei niedriger Temperatur zerfallenden Initiator für den Start der Polymerisation einzusetzen und dann die Hauptpolymerisation mit einem Initiator zu Ende zu führen, der bei höherer Temperatur zerfällt.

Für die im folgenden angegebenen Temperaturbereiche kann man beispielsweise die dafür aufgeführten Initiatoren verwenden.

Temperatur: 40 bis 60°C
Acetylcyclohexansulfonylperoxid, Diacetylperoxidicarbonat, Dicyclohexylperoxidicarbonat, Di-2-ethylhexylperoxidicarbonat, tert.-Butylperneodecanoat, tert.-Amylperneodecanoat, 2,2'-Azobis-(4-methoxy-2,4-dimethylvaleronitril), 2,2'-Azobis-(2-amidinopropan)-dihydrochlorid, 2,2'-Azobis-[2- (2-imidazolin-2-yl)-propan]dihydrochlorid;

Temperatur: 60 bis 80°C
tert.-Butylperpivalat, tert.-Amylperpivalat, Dioctanoylperoxid, Dilaurylperoxid, 2,2'-Azobis(2,4-dimethylvaleronitril);

Temperatur: 80 bis 100°C
Dibenzoylperoxid, tert.-Butylper-2-ethylhexanoat, tert.-Butylpermaleinat, 2,2'-Azobis(isobutyronitril). Dimethyl-2,2'-azobisisobutyrat;

Temperatur: 100 bis 120°C
Bis-(tert.-butylperoxi)-cyclohexan, tert.-Butylperoxiisopropyl-carbonat, tert.-Butylperacetat, Wasserstoffperoxid, tert.-Butylperbenzoat, tert.-Butylper-3,5,5-trimethylhexanoat;

Temperatur: 120 bis 140°C
2,2-Bis-(tert.-butylperoxi)-butan, Dicumylperoxid, Di-tert.-amylperoxid, Di-tert.-butylperoxid;

Temperatur: > 140°C
p-Menthanhydroperoxid, Pinanhydroperoxid, Cumolhydroperoxid und tert.-Butylhydroperoxid.
Bevorzugt sind solche Initiatoren, die bei Raumtemperatur in flüssiger Form vorliegen.

Verwendet man zusätzlich zu den genannten Initiatoren noch Salze oder Komplexe von Schwermetallen, z. B. Kupfer-, Kobalt-, Mangan-, Eisen-, Vanadium-, Nickel- oder Chromsalze, oder organische Verbindungen, z. B. Benzoin, Dimethylanilin oder Ascorbinsäure, so können die Halbwertszeiten der angegebenen radikalbildenden Initiatoren verringert werden. So kann man beispielsweise tert.-Butylhydroperoxid unter Zusatz von 5 ppm Kupfer-acetylacetonat so aktivieren, daß bereits bei 100°C polymerisiert werden kann.

Bezogen auf das bei der Polymerisation eingesetzte Gesamtgewicht der Monomeren verwendet man 0,01 bis 20, vorzugsweise 0,05 bis 10 Gew.-% eines Polymerisationsinitiators oder einer Mischung mehrerer Polymerisationsinitiatoren. Sofern Schwermetalle als Katalysatorbestandteil eingesetzt werden, betragen die angewendeten Mengen 0,1 bis 100 ppm, vorzugsweise 0,5 bis 10 ppm. Oft ist es von Vorteil, eine Kombination aus Peroxid, Reduktionsmittel und Schwermetall als Redoxkatalysator einzusetzen.

Das Molekulargewicht der erfindungsgemäß hergestellten Copolymerisate kann, falls gewünscht, durch Zugabe von Reglern in die Polymerisationsmischung modifiziert, d. h. erniedrigt werden. Als Regler für das Molekulargewicht können Schwefelverbindungen, wie Thioether oder Disulfide, Halogenverbindungen, wie Tetrachlorkohlenstoff oder 1,1,1-Tribrompropan, Ether, wie Tetrahydrofuran und Aldehyde wie z. B. Acetaldehyd oder Butyraldehyd eingesetzt werden.

Durch geeignete Wahl von Reglern, Initiatoren, Polymerisationstemperatur und der Monomerkonzentration, falls die Copolymerisation in einem inerten Löse- oder Verdünnungsmittel durchgeführt wird, wird der K-Wert der erhaltenen Copolymerisate, der ein Maß für das Molekulargewicht ist, eingestellt. Die K-Werte betragen üblicherweise 10 bis 110, vorzugsweise 15 bis 80, wobei die Messungen an 1 gew.-%igen Lösungen je nach Löslichkeit der Copolymerisate entweder in Wasser, Ethanol oder Cyclohexanon vorgenommen werden.

Für die Polymerisation geeignete Apparaturen sind übliche Rührkessel mit Anker-, Blatt-, Impeller- oder Mehrstufenimpulsgegenstromrührer. Gut geeignet sind Apparaturen, die im Anschluß an die Polymerisation die direkte Isolierung des Festproduktes gestatten, wie z. B. Schaufeltrockner.

Die Anhydridgruppen enthaltenden Copolymerisate können einer Vielzahl von polymeranalogen Reaktionen unterworfen werden. So erhält man beispielsweise durch Einwirkung von Alkoholen auf die Anhydridgruppen enthaltenden Copolymerisate aus den Anhydridgruppen die entsprechenden Mono- bzw. Diester. Hierfür kann man beispielsweise zu einer Schmelze der Copolymerisate Methanol, Ethanol, n-Propanol, Isopropanol, n-Butanol, Isobutanol oder Amylalkohol zugeben. Aus den Anhydridgruppen im Copolymerisat erhält man dann die entsprechenden Mono- bzw. Diester. Die Zugabe von Wasser zur Schmelze der Copolymerisate bewirkt eine Hydrolyse der Anhydridgruppen zu Carboxylgruppen. Falls gewünscht, können die Carboxylgruppen der Copolymerisate neutralisiert werden. Hierfür kann man beispielsweise Alkalimetall- und Erdalkalimetallbasen sowie Amine und Ammoniak verwenden. Geeignete Basen sind beispielsweise Natronlauge, Kalilauge, Calciumhydroxyd, Magnesiumoxid, Magnesiumhydroxid, Soda, Natriumhydrogencarbonat, Caliumcarbonat, Caliumhydrogencarbonat oder Amine, wie Triethylamin, Ethanolamin, Diethanolamin, Triethanolamin oder Ammoniak. In Abhängigkeit von den Bedingungen bei der Neutralisation der Anhydridgruppen enthaltenden Copolymerisate werden entweder nur die Anhydridgruppen der Copolymerisate hydrolysiert und neutralisiert oder im stärker alkalischen pH-Bereich auch eine Hydrolyse der Estergruppen der Componenten c) durchgeführt. Dadurch steigt die Konzentration an Carboxylatgruppen im Copolymerisat.

Eine Modifizierung der Anhydridgruppen enthaltenen Copolymerisate mit langkettigen Aminen, wie beispielsweise Stearylamin oder Palmithylamin, unter Bildung von Halbamiden oder Gemischen aus Halbamiden und mit langkettigen Aminen neutralisierten Copolymerisaten gibt Produkte, die insbesondere als Vergrauungsinhibitor in phosphatarmen oder phosphatfreien Waschmitteln von Interesse sind.

Die Anhydridgruppen enthaltenen Copolymerisate können für viele verschiedene Anwendungen eingesetzt werden. In kosmetischen Zubereitungen eignen sie sich in Form alkoholischer Lösungen vor allem als filmbildende Harze in Haarsprays. Als neutralisierte wäßrige Copolymerisatlösungen können sie als Mund- und Zahnpflegemittel gegen Zahnstein, Plaque und Gingivits sowie als Hilfsmittel in Gebißhaftcremes verwendet werden. In Form ihrer Alkalimetall-, Ammonium- oder Erdalkalimetallsalze eignen sich die erfindungsgemäßen Copolymerisate als Verdickungsmittel für wäßrige Systeme, wie kosmetische und pharmazeutische Zubereitungen, Textildruckpasten, Flüssigwaschmittel und Reinigungsmittel. Sie finden außerdem Anwendung als Emulgator, Dispergiermittel, Waschhilfsmittel, Textilschlichte, Wasserbehandlungsmittel und Hilfsmittel bei der Erdölförderung.

Die in den folgenden Beispielen angegebenen K-Werte wurden nach H. Fikentscher an 1 gew-%igen Lösungen in Cyclohexanon oder Wasser bei einem pH-Wert von 7 am Natriumsalz bei 25°C gemessen, vgl. H. Fikentscher, Cellulose-Chemie, Band 13, 58 bis 64 und 71 bis 74 (1932). Die Copolymerisation wurde in sämtlichen Beispielen unter Ausschluß von Sauerstoff in einer Stickstoffatmosphäre durchgeführt.

### Beispiel 1

172 g Maleinsäurediethylester (Zulauf 1), 98 g Maleinsäureanhydrid (Zulauf 2, in einem beheizbaren Tropftrichter), 200 g Vinylisobutylether (Zulauf 3) und 12 g tert.-Butylperneodecanoat (Zulauf 4) werden in entsprechende Dosiergefäße gegeben.

In einem 2 1-Rührbehälter, der mit Rührer, Heizung, Rückflußkühler und den vorbereiteten Dosiervorrichtungen, sowie mit Gasein- und -auslaß ausgestattet ist, werden 111 ml von Zulauf 1, 10 ml von Zulauf 3 und 3 ml von Zulauf 4 vorgelegt und auf 60°C erhitzt. Bei dieser Temperatur werden der restliche Zulauf 1, der restliche Zulauf 3 und der Zulauf 2 in 3 Stunden und der restliche Zulauf 4 in 4 Stunden zudosiert. Anschließend wird noch 1 Stunde bei 80°C gerührt. Man erhält eine farblose hochviskose Schmelze, die bei dieser Temperatur mit 18 g Wasser versetzt und 1 h gerührt wird. Nach Abkühlen auf 75°C werden 480 g Ethanol innerhalb von 15 Minuten zudosiert und 1 h bei dieser Temperatur gerührt. Nach Abkühlen auf 25°C erhält man eine klare, viskose Polymerlösung mit einem Feststoffgehalt von 48,1 Gew.-%. Das Copolymerisat besitzt einen K-Wert von 26,8 (1 gew.-%ig in Cyclohexanon).

### Beispiel 2

172 g Maleinsäurediethylester (Zulauf 1), 98 g Maleinsäureanhydrid (Zulauf 2, in einem beheizbaren Tropftrichter), 200 g Vinylisobutylether (Zulauf 3) und 10 g tert.-Amylperpivalat (Zulauf 4) werden in entsprechende Dosiergefäße gegeben.

In einem 2 1-Rührbehälter, der mit Rührer, Heizung, Rückflußkühler, den mit den Edukten gefüllten Dosiervorrichtungen, Gasein- und -auslaß ausgestattet ist, werden 111 ml von Zulauf 1, 10 ml von Zulauf 3 und 3 ml von Zulauf 4 vorgelegt und auf 80°C erhitzt. Bei dieser Temperatur werden der restliche Zulauf 1, der restliche Zulauf 3 und der Zulauf 2 in 4 Stunden und der restliche Zulauf 4 in 5 Stunden zudosiert. Anschließend wird noch 1 Stunde bei 85°C gerührt. Man erhält eine farblose, hochviskose Schmelze, die bei dieser Temperatur innerhalb 1 Stunde mit 500 g Wasser versetzt wird. Nach Abkühlen auf 25°C erhält man eine klare, viskose Polymerlösung mit einem Feststoffgehalt von 46,3 Gew.-%. Das Copolymerisat besitzt einen K-Wert von 84,3 (1 gew.-%ig in Wasser als Na-Salz).

### Beispiel 3

172 g Maleinsäurediethylester (Zulauf 1), 98 g Maleinsäureanhydrid (Zulauf 2, in einem beheizbaren Tropftrichter), 190 g Vinylisobutylether, 30 g Vinyloctadecylether (beide zusammen Zulauf 3) und 12 g tert.-Butylperneodecanoat (Zulauf 4) werden in entsprechende Dosiergefäße gegeben.

In einem 2 1-Rührbehälter, der wie in Beispiel 1 ausgerüstet ist, werden 111 ml von Zulauf 1, 10 ml von Zulauf 3 und 3 ml von Zulauf 4 vorgelegt und auf 70°C erhitzt. Bei dieser Temperatur werden der restliche Zulauf 1, der restliche Zulauf 3 und Zulauf 2 in 3 Stunden und der restliche Zulauf 4 in 4 Stunden zudosiert. Anschließend wird noch 1 Stunde bei 90°C gerührt. Man erhält eine hellgelbe hochviskose Schmelze, die bei dieser Temperatur mit 18 g Wasser versetzt und 1 Stunde gerührt wird. Nach Abkühlen auf 75°C werden 480 g Ethanol innerhalb von 15 Minuten zudosiert. Das Reaktionsgemisch wird dann 1 h bei dieser Temperatur gerührt. Nach Abkühlen auf 25°C erhält man eine klare viskose Polymerlösung mit einem Feststoffgehalt von 46,0 Gew.-%. Das Copolymerisat hat einen K-Wert von 27,6 (1 gew.-%ig in Cyclohexanon).

### Beispiel 4

172 g Maleinsäurediethylester (Zulauf 1), 98 g Maleinsäureanhydrid (Zulauf 2, in einem beheizbaren Tropftrichter), 195 g Vinylisobutylether, 15 g Vinyloctadecylether (beide zusammen Zulauf 3) und 11 g tert.-Butylper-3,5,5-trimethylhexanoat (Zulauf 4) werden in entsprechende Dosiergefäße gegeben.

In einem 2 1-Rührbehälter, der wie in Beispiel 1 ausgerüstet ist, werden 111 ml von Zulauf 1, 10 ml von Zulauf 3 und 2,5 ml von Zulauf 4 vorgelegt und auf 110°C erhitzt. Bei dieser Temperatur werden der restliche Zulauf 1, der restliche Zulauf 3 und der Zulauf 2 in 6 Stunden und der restliche Zulauf 4 in 7 Stunden zudosiert. Anschließend wird das Reaktionsgemisch noch 1 Stunde bei 125°C gerührt. Man erhält eine gelbliche, viskose Schmelze, die nach Abkühlen auf 75°C innerhalb von 30 Minuten mit 500 g Ethanol versetzt und 1 Stunde gerührt wird. Nach Abkühlen auf 25°C erhält man eine gelbliche, viskose Polymerlösung mit einem Feststoffgehalt von 48,3 Gew.-%. Das Copolymerisat hat einen K-Wert von 34,2 (1 gew.-%ig in Cyclohexanon).

### Beispiel 5

Beispiel 4 wird wiederholt mit den Ausnahmen, daß 12 g tert.-Butylperneodecanoat statt 11 g tert.-Butylper-3,5,5-trimethylhexanoat und daß bei 60°C statt bei 110°C polymerisiert wird. Man erhält eine klare, viskose Polymerlösung mit einem Feststoffgehalt von 47,2 Gew.-%. Das Copolymerisat besitzt einen K-Wert von 29,3 (1 gew.-%ig in Cyclohexanon).

### Beispiel 6

Beispiel 4 wird wiederholt mit den Ausnahmen, daß 15 g Cumolhydroperoxid statt 11 g tert.-Butylper-3,5,5-trimethylhexanoat und daß bei 165°C statt bei 110°C polymerisiert wird. Man erhält eine gelbe, klare, viskose Polymerlösung mit einem Feststoffgehalt von 45,3 Gew.-%. Das Copolymerisat hat einen K-Wert von 28,7 (1 gew.-%ig in Cyclohexanon).

### Beispiel 7

Beispiel 4 wird wiederholt mit den Ausnahmen, daß 15 g tert.-Butylper-2-ethylhexanoat statt 11 g tert.-Butylper-3,5,5-trimethylhexanoat und daß bei 90°C statt bei 110°C polymerisiert wird. Man erhält eine gelbliche, klare, viskose Polymerlösung mit einem Feststoffgehalt von 45,9 Gew.-%. Das Copolymerisat hat einen K-Wert von 36,9 (1 gew.-%ig in Cyclohexanon).

### Beispiel 8

160 g Maleinsäurediethylester (Zulauf 1), 98 g Maleinsäureanhydrid (Zulauf 2, in einem beheizbaren Tropftrichter), 190 g Vinylisobutylether, 30 g Vinyloctadecylether (beide zusammen Zulauf 3) und 12 g 2,2'-Azobis-(2,4-dimethylvaleronitril), gelöst in 12 g Maleinsäurediethylester (Zulauf 4) werden in entsprechende Dosiergefäße gegeben.

In einem 2 1-Rührbehälter, der wie in Beispiel 1 ausgerüstet ist, werden 109 ml von Zulauf 1, 10 ml von Zulauf 3 und 8 ml von Zulauf 4 vorgelegt und auf 80°C erhitzt. Bei dieser Temperatur werden der restliche Zulauf 1, der restliche Zulauf 3 und Zulauf 2 in 4 Stunden und der restliche Zulauf 4 in 5 Stunden zudosiert. Anschließend wird noch 1,5 g 2,2'-Azobis-(2,4-dimethylvaleronitril) zugegeben und 1 Stunde bei 90°C gerührt. Man erhält eine klare, viskose Schmelze, die bei dieser Temperatur mit 18 g Wasser versetzt und 1 Stunde gerührt wird. Nach Abkühlen auf 75°C werden 480 g Ethanol innerhalb von 15 Minuten zudosiert und 1 h bei dieser Temperatur gerührt. Nach Abkühlen auf 25°C erhält man eine klare, viskose Polymerlösung mit einem Feststoffgehalt von 50,3 Gew.-%. Das Copolymerisat hat einen K-Wert von 35,2 (1 gew.-%ig in Cyclohexanon).

### Beispiel 9

86 g Maleinsäurediethylester (Zulauf 1), 49 g Maleinsäureanhydrid (Zulauf 2, in einem beheizbaren Tropftrichter), 297 g Vinyloctadecylether (Zulauf 3, in einem beheizbaren Tropftrichter) und 12 g tert.-Butylperneodecanoat (Zulauf 4) werden in entsprechende Dosiergefäße gegeben.

In einem 2 l-Rührbehälter, der wie in Beispiel 1 ausgerüstet ist, wurden 55 ml von Zulauf 1, 30 ml von Zulauf 3 und 3 ml von Zulauf 4 vorgelegt und auf 60°C erhitzt. Bei dieser Temperatur werden der restliche Zulauf 1, der restliche Zulauf 3 und Zulauf 2 in 3 Stunden und der restliche Zulauf 4 in 4 Stunden zudosiert. Anschließend wird noch 1 Stunde bei 65°C gerührt. Man erhält eine klare viskose Schmelze, die innerhalb von 30 Minuten mit 500 g Ethanol versetzt und 1 Stunde bei 80°C gerührt wird. Nach Abkühlen auf 25°C erhält man eine klare viskose Polymerlösung mit einem Feststoffgehalt von 50,2 Gew.-%. Das Copolymerisat hat einen K-Wert von 16,7 (1 gew.-%ig in Cyclohexanon).

### Beispiel 10

172 g Maieinsäurediethylester (Zulauf 1), 98 g Maleinsäureanhydrid (Zulauf 2, in einem beheizbaren Tropftrichter), 192 g Vinylisobutylether, 25 g Vinyloctadecylether (beide zusammen Zulauf 3) und 12 g tert.-Amylperneodecanoat (Zulauf 4) werden in entsprechende Dosiergefäße gegeben.

In einem 2 l-Rührbehälter, der wie in Beispiel 1 ausgerüstet ist, wurden 111 ml von Zulauf 1, 10 ml von Zulauf 3 und 3 ml von Zulauf 4 vorgelegt und auf 60°C erhitzt. Bei dieser Temperatur werden der restliche Zulauf 1, der restliche Zulauf 3 und Zulauf 2 in 3 Stunden und der restliche Zulauf 4 in 4 Stunden zudosiert. Anschließend wird noch 1 Stunde bei 75°C gerührt. Man erhält eine farblose, hochviskose Schmelze, die bei dieser Temperatur innerhalb 1 Stunde mit 500 g Isopropanol versetzt und nach Beendigung des Isopropanolzulaufes noch 2 Stunden gerührt wird. Nach Abkühlen auf 25°C erhält man eine klare, viskose Lösung mit einem Feststoffgehalt von 47,1 Gew.-%. Das Copolymerisat hat einen K-Wert von 29,1 (1 gew.-%ig in Cyclohexanon).

### Beispiel 11

172 g Maleinsäurediethylester (Zulauf 1), 98 g Maleinsäureanhydrid (Zulauf 2, in einem beheizbaren Tropftrichter), 116 g Vinylmethylether, 45 g Vinyloctadecylether (beide zusammen Zulauf 3) und 12 g tert.-Butylperpivalat (Zulauf 4) werden in entsprechende Dosiergefäße gegeben.

In einem 2 1-Rührbehälter, der wie in Beispiel 1 ausgerüstet ist, wurden 111 ml von Zulauf 1, 10 ml von Zulauf 3 und 3 ml von Zulauf 4 vorgelegt und auf 60°C erhitzt. Bei dieser Temperatur wurden der restliche Zulauf 1, der restliche Zulauf 3 und Zulauf 2 in 3 Stunden und der restliche Zulauf 1, der restliche Zulauf 3 und Zulauf 2 in 3 Stunden und der restliche Zulauf 4 in 4 Stunden zudosiert. Anschließend wird noch 1 Stunde bei 70°C gerührt. Man erhält eine farblose, hochviskose Schmelze, die bei dieser Temperatur zuerst mit 18 g Wasser und anschließend innerhalb 1 Stunde mit 500 g Ethanol versetzt und nach Beendigung des Ethanolzulaufes noch 2 Stunden gerührt wird. Nach Abkühlen auf 25°C erhält man eine klare, viskose Polymerlösung mit einem Feststoffgehalt von 48,4 Gew.-%. Das Copolymerisat hat einen K-Wert von 21,2 (1 gew.-%ig in Cyclohexanon).

### Beispiel 12

172 g Maleinsäurediethylester (Zulauf 1), 98 g Maleinsäureanhydrid (Zulauf 2, in einem beheizbaren Tropftrichter), 110 g Vinylmethylether, 30 g Vinyloctadecylether (beide zusammen Zulauf 3) und 12 g tert.-Butylperneodecanoat (Zulauf 4) wurden in entsprechende Dosiergefäße gegeben.

In einem 2 l-Rührbehälter, der wie in Beispiel 1 ausgerüstet ist, wurden 111 ml von Zulauf 1, 10 ml von Zulauf 3 und 3 ml von Zulauf 4 vorgelegt und auf 60°C erhitzt. Bei dieser Temperatur werden der restliche Zulauf 1, der restliche Zulauf 3 und Zulauf 2 in 3 Stunden und der restliche Zulauf 4 in 6 Stunden zudosiert. Anschließend wird noch 1 Stunde bei 75°C gerührt. Man erhält eine farblose, hochviskose Schmelze, die bei dieser Temperatur zuerst mit 18 g Wasser und anschließend innerhalb 1 Stunde mit 500 g n-Butanol versetzt und nach Beendigung des Alkoholzulaufes noch 2 Stunden gerührt wird. Nach Abkühlen auf 25°C erhält man eine klare, viskose Polymerlösung mit einem Feststoffgehalt von 46,9 Gew.-%. Das Copolymerisat hat einen K-Wert von 22,0 (1 gew.-%ig in Cyclohexanon).

### Beispiel 13

Beispiel 12 wird wiederholt mit der Ausnahme, daß in die Schmelze statt 18 g Wasser und anschließend 500 g n-Butanol, 600 g 10 gew.-%ige Natriumhydroxidlösung gegeben werden. Man erhält eine klare, wäßrige Polymerlösung mit einem Feststoffgehalt von 46,4 Gew.-%, einem pH-Wert von 8,9. Das Copolymerisat hat einen K-Wert von 80,3 (1 gew.-%ig in Wasser).

### Beispiel 14

Beispiel 12 wird wiederholt mit der Ausnahme, daß in die Schmelze statt 18 g Wasser und anschließend 500 g n-Butanol, 74 g Calciumhydroxid gegeben werden. Nach dem Zerkleinern erhält man ein weißes amorphes Pulver, welches als 10 gew.-%ige Lösung einen pH-Wert von 6,5 hat. Das Copolymerisat hat einen K-Wert von 79,1 (1 gew.-%ig in Wasser).

### Beispiel 15

Beispiel 12 wird wiederholt mit der Ausnahme, daß in die Schmelze statt 18 g Wasser und anschließend 500 g n-Butanol, 60 g Isobutylamin bei 70°C zugegeben werden. Man erhält nach Zugabe von 400 g Wasser eine klare, viskose Polymerlösung mit einem Feststoffgehalt von 47,7 Gew.-%. Das Copolymerisat hat einen K-Wert von 57,3 (1 gew.-%ig in Wasser).

### Beispiel 16

206,4 g Maleinsäurediethylester (Zulauf 1), 78,4 g Maleinsäureanhydrid (Zulauf 2, in einem beheizbaren Tropftrichter), 200 g Vinylisobutylether (Zulauf 3) und 12 g tert.-Butyl-2-ethylhexanoat (Zulauf 4) wurden in entsprechende Dosiergefäße gegeben.

In einem 2 l-Rührbehälter, der wie in Beispiel 1 ausgerüstet ist, wurden 111 ml von Zulauf 1, 10 ml von Zulauf 3 und 3 ml von Zulauf 4 vorgelegt und auf 90°C erhitzt. Bei dieser Temperatur wurden der restliche Zulauf 1, der restliche Zulauf 3 und Zulauf 2 in 4 Stunden und der restliche Zulauf 4 in 5 Stunden zudosiert. Anschließend wird noch 1 Stunde bei 100°C gerührt. Man erhält eine farblose, hochviskose Schmelze, die bei dieser Temperatur zuerst mit 14 g Wasser und nach Abkühlen auf 80°C innerhalb einer Stunde mit 500 g Ethanol versetzt und noch 2 Stunden gerührt wurde. Nach Abkühlen auf 25°C erhält man eine klare, viskose Polymerlösung mit einem Feststoffgehalt von 51,3 Gew.-%. Das Copolymerisat hat einen K-Wert von 25,2 (1 gew.-%ig in Cyclohexanon). Es eignet sich als filmbildendes Harz in Haarsprays, vor allem in Pumpsprays.

### Beispiel 17

Beispiel 16 wird wiederholt mit den Ausnahmen, daß statt 206,4 g Maleinsäurediethylester und 78,4 g Maleinsäureanhydrid, 68,8 g Maleinsäurediethylester und 156,8 g Maleinsäureanhydrid verwendet wurden. In die Vorlage kommen statt 111 ml 68,8 g Maleinsäurediethylester. Es werden statt 14 g 29 g Wasser eingesetzt. Man erhält eine klare, viskose Polymerlösung mit einem Feststoffgehalt von 49,2 Gew.-%. Das Copolymerisat hat einen K-Wert von 27,1 (1 gew.-%ig in Cyclohexanon), es kann als filmbildendes Harz in Haarpumpsprays verwendet werden.

### Beispiel 18

Beispiel 16 wird wiederholt mit der Ausnahme, daß statt 206,4 Maleinsäurediethylester, 206,4 g Fumarsäurediethylester eingesetzt wird. Man erhält eine klare, viskose Polymerlösung mit einem Feststoffgehalt von 48,4 Gew.-%. Das Copolymerisat hat einen K-Wert von 20,2 (1 gew.-%ig in Cyclohexanon).

### Beispiel 19

Beispiel 16 wird wiederholt mit der Ausnahme, daß statt 206,4 g Maleinsäurediethylester und 78,4 g Maleinsäureanhydrid, 223,2 g Citraconsäurediethylester und 86,6 g Itaconsäureanhydrid eingesetzt wurden. Man erhält eine klare, viskose Polymerlösung mit einem Feststoffgehalt von 46,9 Gew.-%. Das Copolymerisat hat einen K-Wert von 18,3 (1 gew.-%ig in Cyclohexanon). Es wird als Filmbildner in Haarsprays verwendet.

### Beispiel 20

Beispiel 16 wird wiederholt mit den Ausnahmen, daß statt 206,4 Maleinsäurediethylester und 78,4 g Maleinsäureanhydrid, 242,2 g Glutaconsäuredi-n-butylester und 112,1 g Citraconsäureanhydrid eingesetzt wurden. Es werden statt 14 g 18 g Wasser eingesetzt. Man erhält eine klare, viskose Polymerlösung mit einem Feststoffgehalt von 46,2 Gew.-%. Das Copolymerisat hat einen K-Wert von 19,9 (1 gew.-%ig in Cyclohexanon). Es wird als Filmbildner in Haarpumpsprays verwendet.

### Beispiel 21

Beispiel 16 wird wiederholt mit den Ausnahmen, daß statt 206,4 g Maleinsäurediethylester, 172,8 g Maleinsäuredi-n-hexylester eingesetzt wurden. Man erhält eine klare, viskose Polymerlösung mit einem Feststoffgehalt von 49,5 Gew.-%. Das Copolymerisat hat einen K-Wert von 24,8 (1 gew.-%ig in Cyclohexanon). Es eignet sich als filmbildendes Harz in Haarsprays.

### Beispiel 22

Beispiel 16 wird wiederholt mit der Ausnahme, daß statt 206,4 g Maleinsäurediethylester, 340,8 g Maleinsäuredi-n-hexylester eingesetzt wurden. Man erhält eine viskose Polymerlösung mit einem Feststoffgehalt von 60,3 Gew.-%. Das Copolymerisat hat einen K-Wert von 22,8 (1 gew.-%ig in Cyclohexanon). Es kann als filmbildendes Harz in Haarpumpsprays verwendet werden.

### Beispiel 23

Beispiel 16 wird wiederholt mit den Ausnahmen, daß statt 206,4 g Maleinsäurediethylester und 78,4 g Maleinsäureanhydrid, 364,8 g Maleinsäuredi-n-butylester und 39,2 g Maleinsäureanhydrid eingesetzt wurden. Es wurden statt 14 g 7 g Wasser eingesetzt. Man erhält einen klare, viskose Polymerlösung mit einem Feststoffgehalt von 58,3 Gew.-%. Das Copolymerisat hat einen K-Wert von 17,2 (1 gew.-%ig in Cyclohexanon). Es wird als filmbildendes Harz in Haarpumpsprays in einer Menge von 2 - 20 Gew.-% verwendet.

### Beispiel 24

172 g Maleinsäurediethylester (Zulauf 1), 98 g Maleinsäureanhydrid (Zulauf 2, in einem beheizbaren Tropftrichter), 116 g Vinylmethylether (Zulauf 3, in einem gekühlten Tropftrichter) und 12 g tert.-Butylper-2-ethylhexanoat (Zulauf 4) wurden in entsprechende Dosiergefäße gegeben.

In einem 2 l-Druckreaktor, der mit Rührer, Heizung, Rückflußkühler, den mit den Edukten gefüllten Dosiervorrichtungen, Gasein- und -auslaß ausgestattet ist, werden 111 ml Zulauf 1, 10 ml Zulauf 3 und 3 ml Zulauf 4 vorgelegt und auf 90°C erhitzt. Bei dieser Temperatur und einem Druck von maximal 3,5 bar werden der restliche Zulauf 1, der restliche Zulauf 3 und Zulauf 2 in 3 Stunden und der restliche Zulauf 4 in 4 Stunden zudosiert. Anschließend wird noch 1 Stunde bei 100°C gerührt. Man erhält eine farblose, hochviskose Schmelze, die bei dieser Temperatur innerhalb einer Stunde bei Normaldruck mit 500 g Wasser versetzt und noch 2 Stunden gerührt wird. Nach Abkühlen auf 25°C erhält man eine klare, viskose Polymerlösung mit einem Feststoffgehalt von 44,4 Gew.-%. Das Copolymerisat hat einen K-Wert von 85,6 (1 gew.-%ig in Wasser als Natriumsalz). Es wird als Verdikkungsmittel für wäßrige Systeme im kosmetischen und pharmazeutischen Bereich sowie in Textildruckpasten, Flüssigwaschmitteln und Reinigungsmitteln verwendet.

### Beispiel 25

364,8 g Maleinsäurediethylester (Zulauf 1), 156,8 g Maleinsäureanhydrid (Zulauf 2, in einem beheizbaren Tropftrichter), 80 g Vinylisobutylether (Zulauf 3) und 24 g tert.-Butylper-2-ethylhexanoat (Zulauf 4) werden in entsprechende Dosiergefäße gegeben.

In einem 2 l-Rührbehälter, der wie in Beispiel 1 ausgestattet ist, wurden 111 ml Zulauf 1,8 ml Zulauf 3 und 5 ml Zulauf 4 vorgelegt und auf 90°C erhitzt. Bei dieser Temperatur wurden der restliche Zulauf 1, der restliche Zulauf 3 und der Zulauf 2 in 4 Stunden und der restliche Zulauf 4 in 5 Stunden zudosiert. Anschließend wurde noch 1 Stunde bei 95°C gerührt. Man erhält eine hellgelbe, hochviskose Schmelze, die bei dieser Temperatur zuerst mit 29 g Wasser und nach Abkühlen auf 80°C innerhalb einer Stunde mit 600 g Ethanol versetzt und noch 2 Stunden gerührt wurde. Nach Abkühlen auf 25°C erhält man eine klare, viskose Polymerlösung mit einem Feststoffgehalt von 52,1 Gew.-%. Das Copolymerisat hat einen K-Wert von 15,8 (1 gew.-%ig in Cyclohexanon). Es wird als Filmbildner in Haarpumpsprays in Mengen von 2 - 20 Gew.-% verwendet.

### Beispiel 26

Von der wäßrigen Polymerlösung, die gemäß Beispiel 13 erhalten wurde, wurden nach Zugabe von 20 g Natriumhydroxid ca. 90 g Ethanol durch azeotrope Destillation abgetrennt. Man erhält eine klare, wäßrige Polymerlösung mit einem Feststoffgehalt von 57,3 Gew.-%, einem pH-Wert von 11,6. Das Copolymerisat hat einen K-Wert von 83,4 (1 gew.-%ig in Wasser). Es wird als Verdickungsmittel für wäßrige Systeme im Pharma- und Kosmetiksektor verwendet. In Zusammensetzungen mit Bakteriziden kann es in der Zahnkosmetik als Plaque- und Zahnsteinverhinderer und gegen Gingivitis eingesetzt werden. Ebenso kann es in Flüssigwasch- und Reinigungsmitteln verwendet werden.

## Patentansprüche

1. Copolymerisate auf Basis von Vinylethern und monoethylenisch ungesättigten Dicarbonsäureanhydriden, dadurch gekennzeichnet, daß sie
(a) 2 bis 50 mol.-% Vinylalkylether mit 3 bis 30 C-Atomen im Molekül,
(b) 10 bis 88 mol.-% monoethylenisch ungesättigte Dicarbonsäureanhydride und
(c) 10 bis 88 mol.-% Dialkylester von monoethylenisch ungesättigten Dicarbonsäuren copolymerisisert enthalten.

2. Verfahren zur Herstellung von Copolymerisaten nach Anspruch 1, dadurch gekennzeichnet, daß man
(a) 2 bis 50 mol.-% Vinylalkylether mit 3 bis 30 C-Atomen im Molekül,
(b) 10 bis 88 mol.-% monoethylenisch ungesättigte Dicarbonsäureanhydride und
(c) 10 bis 88 mol.-% Dialkylester von monoethylenisch ungesättigten Dicarbonsäuren
bei Temperaturen von 40 bis 180°C in Gegenwart von Initiatoren copolymerisiert, die unter den Bedingungen der Copolymerisation Radikale bilden.

3. Verwendung der Copolymerisate nach Anspruch 1 als filmbildende Harze in Haarsprays.

4. Verwendung der Copolymerisate nach Anspruch 1 in Form der Alkalimetall-, Ammonium- oder Erdalkalimetallsalze als Verdickungsmittel für wäßrige Systeme, wie kosmetische und pharmazeutische Zubereitungen, Textildruckpasten, Flüssigwaschmittel und Reinigungsmittel.

## Claims

1. A copolymer based on vinyl ethers and monoethylenically unsaturated dicarboxylic anhydrides, which contains
(a) from 2 to 50 mol % of vinyl alkyl ethers having 3 to 30 carbon atoms in the molecule,
(b) from 10 to 88 mol % of monoethylenically unsaturated dicarboxylic anhydrides and
(c) from 10 to 88 mol % of dialkyl esters of monoethylenically unsaturated dicarboxylic acids
as copolymerized units.

2. A process for the preparation of a copolymer as claimed in claim 1, wherein
(a) from 2 to 50 mol % of vinyl alkyl ethers having 3 to 30 carbon atoms in the molecule,
(b) from 10 to 88 mol % of monoethylenically unsaturated dicarboxylic anhydrides and
(c) from 10 to 88 mol % of dialkyl esters of monoethylenically unsaturated dicarboxylic acids
are copolymerized at from 40 to 180°C in the presence of initiators which form free radicals under the copolymerization conditions.

3. Use of a copolymer as claimed in claim 1 as a film-forming resin in hair sprays.

4. Use of a copolymer as claimed in claim 1 in the form of the alkali metal, ammonium or alkaline earth metal salt as a thickener for aqueous systems, such as cosmetic and pharmaceutical formulations, textile print pastes, liquid detergents and cleaning agents.

## Revendications

1. Copolymères à base d'éthers vinyliques et d'anhydrides d'acides dicarboxyliques monoéthyléniquement insaturés, caractérisés en ce qu'ils contiennent, à l'état copolymérisé
(a) 2 à 50% molaires d'éthers alkylvinyliques qui comportent de 3 à 30 atomes de carbone dans la molécule,
(b) 10 à 88% molaires d'anhydrides d'acides dicarboxyliques monoéthyléniquement insaturés, et
(c) 10 à 88% molaires d'esters dialkyliques d'acides dicarboxyliques monoéthyléniquement insaturés.

2. Procédé de préparation de copolymères suivant la revendication 1, caractérisé en ce que l'on copolymérise
(a) 2 à 50% molaires d'éthers alkylvinyliques qui comportent de 3 à 30 atomes de carbone dans la molécule,
(b) 10 à 88% molaires d'anhydrides d'acides dicarboxyliques monoéthyléniquement insaturés, et
(c) 10 à 88% molaires d'esters dialkyliques d'acides dicarboxyliques monoéthyléniquement insaturés,
à des températures de 40 à 180°C, en présence d'amorceurs qui engendrent des radicaux dans les conditions de la copolymérisation.

3. Utilisation des copolymères suivant la revendication 1, à titre de résines filmogènes dans des sprays capillaires.

4. Utilisation des copolymères suivant la revendication 1, sous la forme des sels de métaux alcalins, d'ammonium, ou de métaux alcalino-terreux, à titre d'agents épaississants pour des systèmes aqueux, comme des compositions cosmétiques et pharmaceutiques, des pâtes d'impression pour matières textiles, des agents de nettoyage et de lavage liquides.
